(19) Europäisches Patentamt
European Patent Office
Office européen des brevets

(11) **EP 1 250 895 B1**

(12) **EUROPÄISCHE PATENTSCHRIFT**

(45) Veröffentlichungstag und Bekanntmachung des Hinweises auf die Patenterteilung:
**12.12.2007 Patentblatt 2007/50**

(51) Int Cl.:
*A61C 13/00* (2006.01)     *A61C 5/10* (2006.01)
*A61C 13/083* (2006.01)     *C25D 1/14* (2006.01)

(21) Anmeldenummer: **02008348.1**

(22) Anmeldetag: **12.04.2002**

(54) **Verfahren zur Herstellung vollkeramischer Dentalformteile**

Method for producing dental ceramic elements

Méthode pour obtenir des pièces dentaires en céramique

(84) Benannte Vertragsstaaten:
**AT BE CH CY DE DK ES FI FR GB GR IE IT LI LU MC NL PT SE TR**

(30) Priorität: **18.04.2001 DE 10120084**

(43) Veröffentlichungstag der Anmeldung:
**23.10.2002 Patentblatt 2002/43**

(73) Patentinhaber: **Wieland Dental + Technik GmbH & Co. KG**
**75179 Pforzheim (DE)**

(72) Erfinder:
• **Laubersheimer, Jürgen**
**76307 Karlsbad (DE)**
• **Knoll, Stefan**
**70195 Stuttgart (DE)**
• **Burger, Goran**
**75179 Pforzheim (DE)**

(74) Vertreter: **Patentanwälte**
**Ruff, Wilhelm, Beier, Dauster & Partner**
**Kronenstrasse 30**
**70174 Stuttgart (DE)**

(56) Entgegenhaltungen:
WO-A-01/85053     WO-A-99/35994
WO-A1-01/12097     WO-A1-99/50480
DE-A1- 2 705 770     DE-C1- 19 816 546

**Beschreibung**

Die Erfindung betrifft ein Verfahren zur Herstellung vollkeramischer Dentalformteile wie Kronen, Brükken o. dgl. sowie ein mit diesem Verfahren herstellbares vollkeramisches Dentalformteil.

Schon immer war Keramik oder "Porzellan" ein attraktiver Werkstoff, um Zähne mit sehr zahnähnlichem Aussehen in Form und Farbe nachzubilden. Keramik ist ein chemisch beständiger, korrosionsfester und biokompatibler Werkstoff, der zudem noch in schier unendlicher Menge in mineralischer Form verfügbar und somit preiswert ist. Aus diesem Werkstoff ist mit zahntechnischen Mitteln individueller Zahnersatz einfach und reproduzierbar herzustellen, so daß der Durchbruch des Werkstoffes "Dentalkeramik" eingetreten ist.

Um die einzige Schwäche dieses Werkstoffes, die Sprödigkeit, zu umgehen, wird zahntechnisch gefertigter Zahnersatz in der Regel schon seit langem als klassischer Werkstoff-Verbund hergestellt, z. B. als sogenannte Metallkeramik. Eine metallkeramische Krone oder Brücke besteht aus einem metallischen Gerüst bzw. Unterbau und einer der Zahnform nachempfundenen sogenannten Verblendung aus Dentalkeramik. Der Unterbau wird beim Einsetzen des Zahnersatzes direkt auf dem nach der zahnärztlichen Präparation verbleibenden Restzahn befestigt und wird oft als (Schutz-)Käppchen bezeichnet. Je nachdem, aus welchem Metall bzw. aus welcher Legierung die Käppchen bestehen und je nach Herstellungsverfahren (Gießen, Galvanoforming-Verfahren, d. h. galvanische Abscheidung), können Probleme in Form von Korrosion und daraus resultierende Verfärbungen, Körperunverträglichkeiten u.a.m. entstehen. Deshalb wurden in den letzten Jahren zunehmend Systeme entwickelt, die vergleichbare Unterkonstruktion aus keramischen Materialien herstellen und zahntechnisch weiterverarbeiten können.

Es gibt bereits mehrere funktionierende Systeme auf dem Dentalmarkt. So werden die Keramik-Käppchen beispielsweise durch manuelles Auftragen eines Schlickers auf einen Modellstumpf, anschließendem Sinterbrand sowie nachfolgender Infiltration mit Spezialglas (VITA In-Ceram) oder durch einen Pressvorgang unter Temperatureinwirkung (Empress, Fa. IVOCLAR) hergestellt. Es gibt auch Systeme, bei denen die Käppchen aus vorgesinterten Keramikblöcken digital gefräst werden (DCS-Systeme, CEREC usw.). Allen solchen sogenannten Vollkeramik-Systemen ist jedoch gemeinsam, daß sie die Paßgenauigkeit metallischer Käppchen auf dem Restzahn, ob letztere nun gegossen sind oder durch galvanische Prozesse entstehen, in der Regel nicht erreichen.

Die mangelnde Paßgenauigkeit existierender Vollkeramik-Systeme ergibt sich durch die verwendeten Formgebungsverfahren. Bei der Herstellung metallischer Käppchen wird gegossen oder galvanisiert, so daß sich das Metall in geschmolzener bzw. gelöster Form optimal der Stumpfgeometrie anpassen kann. Dagegen muß z. B. bei CADCAM-gestützten Vollkeramikverfahren auch diese dem Stumpf zugewandte Oberfläche nach einem digital aufgenommenen Datensatz aus festem Material spanabhebend gefräst werden.

Sowohl in der DE 198 16 546 C1 als auch in der WO 99/35994 A1 ist ein Verfahren zur Herstellung von Zahnersatz offenbart, bei dem ein Schlicker aus Keramikmaterial auf ein Arbeitsmodell aufgebracht und dieses aufgebrachte Schlickermaterial anschließend auf eine vorgegebene Stärke abgefräst wird. Beim Auftrag des Schlickers ist das Arbeitsmodell auf der Welle eines Motors einer CNC-Fräsmaschine befestigt und in Drehbewegung versetzt. Der Schlickerauftrag selbst erfolgt mit Hilfe eines Pinsels oder einer das Keramikmaterial lokal aufbringende Auftragsvorrichtung (Auftragspipette). Alternative Auftragsmöglichkeiten für den Schlicker sind nicht angesprochen.

In der WO 99/50480 A1 ist ein Verfahren zur elektrophoretischen Abscheidung von keramischen Partikeln zur Herstellung eines Grünkörpers für einen Zahnersatz beschrieben. Eine anschließende Bearbeitung des erhaltenen keramischen Körpers ist dort nicht angesprochen. Der keramische Körper wird nach einem Vorsinterschritt glasinfiltriert.

Die WO 01/12097 A1 schließlich befasst sich mit der Herstellung von Zahnersatz durch Herausfräsen aus einem vorgesinterten Rohling und anschließendem Dichtsintern. Das Aufbringen von keramischen Teilchen aus einem Schlicker auf ein Arbeitsmodell hat mit der in der WO 01/12097 A1 dargestellten Vorgehensweise keine Berührungspunkte.

Die Erfindung stellt sich die Aufgabe, die beschriebenen und weitere Nachteile des Standes der Technik zu vermeiden. Es soll ein neues Verfahren zur Herstellung vollkeramischer Dentalformteile bereitgestellt werden, bei dem die erhaltenen Formteile eine hohe, vorzugsweise höhere Passgenauigkeit mit den Grundstrukturen, für die sie vorgesehen sind, aufweisen.

Diese Aufgabe wird gelöst durch das Verfahren mit den Merkmalen des Anspruchs 1. Bevorzugte Ausführungsformen dieses Verfahrens sind in den abhängigen Ansprüchen 2 bis 34 dargestellt. Der Wortlaut sämtlicher Ansprüche wird hiermit durch Bezugnahme zum Inhalt der Beschreibung gemacht.

Bei dem erfindungsgemäßen Verfahren wird zunächst eine Suspension keramischer Partikel auf ein Modell der Grundstruktur, für die das Dentalformteil vorgesehen ist, aufgebracht. Dann wird zumindest die äußere Oberfläche des erhaltenen keramischen Körpers (Grünkörpers), die dem Modell und damit später der Grundstruktur abgewandt ist, mindestens teilweise unter Abtrag von keramischem Material bearbeitet. Gegebenenfalls erfolgt diese Bearbeitung, nachdem der keramische Grünkörper vom Modell entfernt worden ist.

Das Aufbringen der Suspension und damit der keramischen Partikel auf das Modell erfolgt durch elektrophoretische Abscheidung. Die Grundlagen und die Durchführung einer solchen elektrophoretischen Abscheidung sind dem Fachmann bekannt. Dabei wird in Flüssigkeit dispergiertes, in diesem Fall keramisches Pulver, mit Hilfe eines elektrischen Feldes auf dem Modell/Arbeitsmodell als bereits vorverdichtete Schicht abgeschieden.

Die oben erwähnte Bearbeitung erfolgt vorzugsweise mit Hilfe von Verfahren, die üblicherweise als "spanabhebend" bezeichnet werden. Ein besonders hervorzuhebendes solches Verfahren ist das Fräsen. Dieses Fräsen kann wie alle anderen verwendbaren Verfahren vorzugsweise CADCAM-gestützt erfolgen. Insoweit kann auch auf den eingangs beschriebenen entsprechenden Stand der Technik verwiesen werden.

Die unter Abtrag von keramischem Material erfolgende Bearbeitung kann bei bevorzugten Ausführungen der Erfindung im Grünzustand erfolgen, Dies bedeutet, dass der keramische Körper unmittelbar nach seiner Ausbildung ohne weiteren Sinterschritt, vorzugsweise durch Fräsen, bearbeitet wird. Bei anderen bevorzugten Ausführungsformen kann diese Bearbeitung jedoch im mindestens teilweise gesinterten Zustand des keramischen Körpers erfolgen. Ein solcher vorgeschalteter Sinterschritt hat beispielsweise den Vorteil, dass der keramische Körper bei der Vorbereitung und Durchführung der Bearbeitung, z.B. dem Fräsen, besser handhabbar ist.

Bei der Erfindung werden also Oberflächen des Dentalformteils auf unterschiedliche Weise hergestellt. Durch das Aufbringen der Suspension keramischer Partikel (des keramischen Schlickers) wird die innere Oberfläche des Formteils definiert. Dies ist diejenige, die der dentalen Grundstruktur, also beispielsweise dem Zahnstumpf oder einem Implantataufbauteil zugewandt ist. Durch dieses Formgebungsverfahren, insbesondere die elektrophoretische Abscheidung, wird hier eine besonders gute Passung zwischen dieser inneren Oberfläche und der Grundstruktur erreicht. Die äußere Oberfläche des Dentalformteils wird durch die nachfolgende Bearbeitung, insbesondere den CADCAM-gestützten Fräsvorgang definiert. Hier handelt es sich um die Oberfläche, die später der Grundstruktur abgewandt ist und gegebenenfalls mit einer Verblendung (Kunststoff, Keramik) versehen wird. Damit werden durch die Erfindung die Vorteile eines direkten Aufbringens (elektrophoretische Abscheidung) und einer mechanischen Bearbeitung (CADCAM-gestütztes Fräsen) kombiniert.

Zweckmäßigerweise werden die keramischen Partikel in einer Schichtdicke auf das Modell der Grundstruktur aufgebracht, die die spätere Bearbeitung berücksichtigt. Der erhaltene keramische Körper (Grünkörper) ist also aus-reichend dick, damit die unter Abtrag erfolgende Nachbearbeitung möglich ist. Andererseits wird die Dicke des Grünkörpers so gewählt werden, daß bei der Nachbearbeitung nicht zuviel Material abgetragen werden muß, um das Gesamtverfahren insgesamt ökonomisch zu gestalten. Nach seiner Bearbeitung wird das Dentalformteil (vor einer möglichen Verblendung) üblicherweise eine Schichtdicke zwischen 0,2 und 0,8 mm, vorzugsweise zwischen 0,4 und 0,6 mm aufweisen.

Bei der Erfindung kann die Suspension vorzugsweise keramische Fasern, nanokristalline Partikel und/oder metallisches Pulver enthalten.

Der Anteil der keramischen Fasern an der Gesamtmenge der Keramik-partikel ist vorzugsweise relativ gering. Er beträgt beispielsweise ca. 0,5 Gewichtsprozent bis 5 Gewichtsprozent, insbesondere 1 Gewichtsprozent bis 2 Gewichtsprozent. Der keramische Schlicker besteht also vorzugsweise hauptsächlich aus keramischen Pulverpartikeln, im folgenden Primärpartikel genannt, und einem geringen Anteil an keramischen Fasern. Die Primärpartikel besitzen einen mittleren Durchmesser, der im Bereich von 0,4 $\mu$m bis 0,8 $\mu$m, vorzugsweise 0,6 $\mu$m bis 0,7 $\mu$m liegt. Der mittlere Durchmesser der keramischen Fasern kann deutlich größer sein. Vorzugsweise liegt er im Bereich von 1 um bis 20 $\mu$m, insbesondere 5 $\mu$m bis 10 $\mu$m. Die Länge der keramischen Fasern kann im Bereich von 0,1 mm bis 50 mm, insbesondere 5 mm bis 10 mm liegen. Besonders bevorzugt handelt es sich bei den keramischen Fasern, wie bei den Primärpartikeln, um solche aus der Gruppe der Oxidkeramiken. Die Primärpartikel bzw. Fasern können beispielsweise aus Aluminiumoxid und/oder Zirkoniumoxid bestehen. Das Zirkoniumoxid kann gegebenenfalls mit Yttriumoxid, Ceriumoxid o. dgl. stabilisiert sein.

Alternativ zu den keramischen Fasern sind keramische Schlicker mit nanokristallinen Partikeln einsetzbar. Es ist jedoch auch möglich, keramische Fasern und nanokristalline Partikel einzusetzen, die gemeinsam abgeschieden werden.

Der Anteil der nanokristallinen Partikel an der Gesamtmenge der Keramikpartikel beträgt vorzugsweise 0,5 Gewichtsprozent bis 10 Gewichtsprozent, insbesondere 1 Gewichtsprozent bis 2 Gewichtsprozent. Die nanokristallinen Partikel sind im Vergleich zu den keramischen Primärpartikeln sehr viel kleiner. Der mittlere Durchmesser der nanokristallinen Partikel kann im Bereich von 1 nm bis 200 nm, insbesondere 5 nm bis 20 nm liegen. Wie die keramischen Primärpartikel, handelt es sich bei den nanokristallinen Partikeln vorzugsweise um solche aus der Gruppe der Oxidkeramiken. Die nanokristallinen Partikel können beispielsweise aus Aluminiumoxid und/oder Zirkoniumoxid sein.

Der Anteil des metallischen Pulvers an der Gesamtmenge der disper-gierten Partikel kann in großen Bereichen schwanken. Er beträgt beispielsweise ca. 10 Gewichtsprozent bis 80 Gewichtsprozent, vorzugsweise 30 Gewichtsprozent bis 60 Gewichtsprozent. Der dispergierte Anteil im keramischen Schlicker besteht zum Teil, vorzugsweise etwa zu einem Drittel, aus metallischem Pulver. Der Rest wird von keramischen Pulverpartikeln gebildet, die im folgenden Primärpartikel genannt werden. Die Primärpartikel besitzen einen mittleren Durchmesser, der im Bereich von 0,4 $\mu$m bis 0,8 $\mu$m, vorzugsweise 0,6 $\mu$m bis 0,7 $\mu$m liegt. Der mittlere Durchmesser der metallischen Pulverpartikel kann deutlich größer sein. Vor-zugsweise liegt er im Bereich von 1 $\mu$m bis 50 $\mu$m, vorzugsweise 2 $\mu$m bis 10 $\mu$m.

Um eine Oxidation des metallischen Pulvers bei dem oxidativen Sintervorgang zu gewährleisten, handelt es sich beim metallischen Pulver vorzugsweise um ein Pulver aus Nicht-Edelmetall. Dabei kann es sich bei dem Metallpulver um ein Metall handeln, dessen Metallionen im keramischen Pulver enthalten sind. Nach dem Sintervorgang, bei dem das Metallpulver oxidiert wird, sind die nunmehr gebildeten Metalloxidpartikel praktisch nicht mehr von den keramischen Primärpartikeln unterscheidbar. Der dentale Formkörper besteht also aus einem homogenen oxidischen Gefüge. Als Metallpulver kann beispielsweise Aluminium- und/oder Zirkoniumpulver eingesetzt werden.

Aus dem keramischen Pulver und dem metallischen Pulver kann eine im wesentlichen homogene Mischung hergestellt werden. Die Mischung kann z. B. durch Mischmalen der beiden Pulver im festen Zustand hergestellt werden. Dabei kann der mittlere Durchmesser der metallischen Pulverpartikel verkleinert werden.

Erfindungsgemäß wird eine Suspension, die in mindestens einem Lösungsmittel keramische Partikel, mindestens ein Dispergierungsmittel und gegebenenfalls weitere Additive, beispielsweise Bindemittel enthält, zur Herstellung vollkeramischer Dentalformteile verwendet. Dabei handelt es sich bei dem Dispergierungsmittel um eine Verbindung, die mindestens eine Carboxylgruppe aufweist.

Zum besseren Verständnis der Erfindung werden im folgenden einige Ausführungen zu Suspensionen keramischer Partikel, die auch als keramische Schlicker bezeichnet werden, gemacht.

Unter keramischen Schlickern versteht man Suspensionen dispergierter keramischer Pulver in geeigneten Lösungsmitteln, meist Wasser oder einfache Alkohole. Dabei werden Suspensionen möglichst hoher Feststoffgehalte bei möglichst niedriger Viskosität angestrebt. Um eine schnelle Sedimentation der Partikel zu verhindern, werden bei der Herstellung der Schlicker meist Additive wie Dispergierungsmittel und sogenannte Binder in kleinen Mengen zugegeben.

Feine Pulver mit großen Oberflächen neigen aufgrund der starken Van-der-Waals-Kräfte, die zwischen den Pulverpartikeln wirken, stark zur Agglomeration. Zur Ausnutzung des Potentials der feinen Pulver im Hinblick auf hohe mechanische Festigkeiten, niedrige Brenntemperaturen und hohe Paßgenauigkeiten der keramischen Gerüstelemente, ist eine Kontrollierung der interpartikulären Anziehungskräfte zwischen den Pulverpartikeln während der Verarbeitung notwendig. Eine weitgehend agglomeratfreie Pulververarbeitung ist nur in Form von kolloidalen Suspensionen gewährleistet. Dabei fällt der Dispergierung der Pulver eine entscheidende Rolle zu, weil die anschließenden Verfahrensschritte Formgebung und Sinterung maßgeblich von dem Dispergierzustand der Schlicker bestimmt werden.

Ziel der Dispergierung ist es, mit Hilfe von physikochemischen und mechanischen Mitteln den genannten Kräften so entgegenzuwirken, daß eine Suspension resultiert, in der die Pulverpartikel stabil im deagglomerierten Zustand vorliegen. Die maßgeblichen Grundprozesse hierfür sind neben der Benetzung des Feststoffes, die Deagglomerierung der Partikelzusammenlagerungen sowie die Stabilisierung der dispergierten Suspensionen. Eine erfolgreiche Dispergierung ist damit mit bestimmten Randbedingungen verknüpft. So sollte beispielsweise das Dispergiermedium so gewählt sein, daß das Lösungsmittel den Feststoff möglichst spontan und vollständig benetzt und dabei jedoch den Feststoff weder löst noch chemisch verändert. Darüber hinaus sollte zwischen den Pulverpartikeln eine möglichst hohe Abstoßungsenergie erreicht werden.

Von entscheidender Bedeutung für den resultierenden Dispergierzustand des Schlickers sind vor allem die in dem flüssigen Medium erreichbaren abstoßenden Kräfte zwischen den einzelnen Pulverpartikeln. Grundlage hierfür ist häufig ein mechanisches Vorbehandlungsverfahren, das einen möglichst hohen Energieeintrag und damit eine vollständige Deagglomeration gewährleistet. Es kommen neben hochtourigem Rühren hauptsächlich Mahlen oder eine Ultraschallbehandlung in Betracht. Die wirkungsvollste und effektivste Methode zur Dispergierung stellt dabei die Ultraschallbehandlung dar. Um ein erneutes Agglomerieren zu verhindern, werden Dispergierhilfen eingesetzt, die die anziehenden Kräfte zwischen den Partikeln reduzieren und entweder elektrostatisch oder sterisch stabilisierend wirken. Im Falle der sterischen Stabilisierung verhindern Polymerketten, die die Pulveroberflächen belegen, eine Annäherung der Pulverpartikel. Höhere Abstoßungsenergien werden mit Hilfe von elektrostatischen Abstoßungskräften erhalten. Hierbei werden durch ober-flächenaktive Substanzen, wie z. B. anorganische oder organische Säuren und Basen, sowohl in wäßrigen als auch in organischen Lösungsmitteln Ladungen auf den Pulveroberflächen erzeugt. Die Oberflächenladungen bauen in dem Dispergiermedium eine Hülle entgegengesetzt geladener Ionen um sich auf, die die Partikel nach außen neutral erscheinen lassen. Durch die Brown'sche Molekularbewegung der dispergierten Partikel in dem Schlicker wird ein Teil dieser Doppelschicht mitgezogen, wodurch an der Scherebene eine Potentialdifferenz entsteht. Diese Potentialdifferenz ist als sogenanntes Zeta-Potential experimentell über die Partikelwanderungsgeschwindigkeit im elektrischen Feld bestimmbar. Hierbei gelten näherungsweise nach der SMOLUCHOWSKY-Gleichung folgende Zusammenhänge:

$$\text{(Gl. 1)} \qquad \mu = v/E = \varepsilon\, \xi\, /\, 4\, \pi\, \eta$$

Darin ist die auf die elektrische Feldstärke E bezogene Partikelwanderungsgeschwindigkeit v [m/s] die elektrophoretische Beweglichkeit $\mu$ [m²/V*s], $\varepsilon$ die Dielektrizitätskonstante des flüssigen Medium, $\eta$ die dy-

namische Viskosität und ξ das elektrokinetische Potential oder Zeta-Potential. Das Zeta-Potential hängt von Form, Oberflächenbeschaffenheit und Leitfähigkeit der Pulverpartikel ab und läßt sich durch folgende Gleichung beschreiben:

$$(\text{Gl. 2}) \qquad \xi = 4\,\pi\,v\,\eta\,/\,\varepsilon\,E$$

Die elektrostatischen Abstoßungskräfte zwischen den Pulverpartikeln sind damit bei einem maximalen Betrag von ξ am größten. Grundvoraussetzung für eine agglomeratfreie Verarbeitung in kolloidalen Suspensionen ist damit der gezielte Einsatz von Dispergierhilfen, die eine hohe Oberflächenladung auf den Partikeln induzieren und somit die Ausbildung eines maximalen Zeta-Potentials ermöglichen.

Für die weitere Erläuterung der Erfindung sei hier ebenfalls kurz die Herstellung und Weiterverarbeitung zahntechnischer Modelle erläutert. Der Zahn oder die Zähne, die mit dem Dentalformteil versehen werden sollen, werden vom Zahnarzt in entsprechender Weise präpariert. Von dieser Mundsituation entnimmt der Zahnarzt einen Abdruck mit Hilfe eines aushärtenden Elastomermaterials. Hier kann es sich beispielsweise um einen Silikonkunststoff handeln. Der so erhaltene Abdruck stellt ein Negativmodel der vom Zahnarzt vorgenommenen Präparation dar. Dieser Abdruck, d. h. das Negativmodell, wird dem Zahntechniker übergeben, der diesen Abdruck mit Hilfe eines geeigneten Modellmaterials, meist Dentalgips, ausgießt. Nach dem Abbinden des Gipses entsteht ein Positivmodell, das sogenannte Meistermodell, welches der Präparation des Zahnarztes exakt entspricht. Dieses Meistermodell wird üblicherweise als Vorlage zurückbehalten. Es wird dazu verwendet, ein oder mehrere Arbeitsmodelle herzustellen, die dann weiterverarbeitet werden. Die Herstellung des Arbeitsmodells erfolgt durch Duplieren, d. h. mit Hilfe eines Dupliermaterials, beispielsweise Silikonkunststoff, wird eine Negativmodell hergestellt, das dann wiederum mit Gips ausgegossen wird. Auf diese Weise wird ein weiteres Positivmodell, nämlich das Arbeitsmodell, erstellt.

Bei bevorzugten Ausführungsformen der Erfindung werden die vollkeramischen Dentalformteile, wie bereits erwähnt, durch elektrophoretische Abscheidung hergestellt. Bei der elektrophoretischen Formgebung wird das Modell der Mundsituation, das elektrisch z. B. mit Leitsilberlack kontaktiert ist, als Elektrode in einen Stromkreis geschalten. Als Gegenelektrode dient beispielsweise eine Platin-Elektrode, deren Form je nach Form des Modells variiert werden kann, um ein hohes homogenes elektrisches Feld für das gesamte Modell zu erreichen.

Die Abscheidung mit dem hergestellten Schlikker auf das Arbeitsmodell erfolgt bei konstant gehaltener Spannung bzw. bei konstant gehaltenem Strom über einen Zeitraum von 1 bis 60 Minuten. Typische Werte für die Abscheidespannung bzw. Abscheideströme liegen zwischen 1 und 100 V bzw. zwischen 0 und 500 mA. Die bei Verwendung der elektrophoretischen Abscheidung erhaltenen Gründichten sind üblicherweise größer als 70 %, vorzugsweise größer als 80 % der theoretischen Dichte. Die elektrophoretische Abscheidung kann gegebenenfalls automatisiert mit Hilfe eines entsprechenden Geräts erfolgen.

Die erfindungsgemäß verwendeten Suspensionen keramischer Partikel sind wie ausgeführt Suspensionen dispergierter keramischer Pulver in geeigneten Lösungsmitteln. Als Lösungsmittel werden vorzugsweise polare Lösungsmittel verwendet, wobei es sich vorzugsweise um Wasser, Alkohole und deren Mischungen oder Mischungen aus Wasser mit Alkoholen handelt. Vorzugsweise werden polare Lösungsmittel mit Dielektrizitätszahlen im Bereich zwischen 15 und 85, vorzugsweise im Bereich von 15 bis 20 verwendet.

Bei den keramischen Partikeln handelt es sich vorzugsweise um oxidkeramische Partikel, insbesondere um Aluminiumoxid ($Al_2O_3$)-Partikel und/oder Zirkonoxid ($ZrO_2$)-Partikel oder deren Mischungen. Die Korngrößen der keramischen Partikel liegen vorzugsweise zwischen 1 nm und 100 $\mu$m, vorzugsweise zwischen 100 nm und 10 $\mu$m. Insbesondere sind die keramischen Partikel in der Suspension in einer Menge zwischen 10 und 90 Gewichtsprozent, vorzugsweise zwischen 40 und 60 Gewichtsprozent, bezogen auf das Gesamtgewicht der Suspension, enthalten.

Üblicherweise sind noch Bindemittel Bestandteil der Suspension, wobei es sich vorzugsweise um mindestens ein Polyvinylbutyral handelt. Solche Bindemittel dienen u. a. zur Verbesserung sowohl des Trocknungsverhaltens als auch der Festigkeiten der resultierenden Grünkörper. Die Bindemittel sind in der Suspension, bezogen auf deren Feststoffgehalt vorzugsweise in Mengen zwischen 0,1 und 20 Gewichtsprozent, insbesondere zwischen 0,2 und 2 Gewichtsprozent enthalten.

Die verwendeten Schlicker zeichnen sich durch Viskositäten im Bereich von 1 mPa*s bis 50 mPa*s, vorzugsweise im Bereich von 3 bis 10 mPa*s bei einer Scherrate von 600 s$^{-1}$ aus. Die durch die zugegebenen Dispergierhilfe erhaltenen Zeta-Potentiale der Schlicker liegen zwischen ± 1 mV und ± 100 mV, vorzugsweise zwischen ± 30 mV und ± 50 mV.

Nach der Erfindung sind Ausführungsformen besonders bevorzugt, bei denen in der keramischen Suspension bestimmte Dispergierungsmittel eingesetzt werden. In diesen Fällen handelt es sich bei dem Dispergierungsmittel um mindestens eine Carbonsäure, vorzugsweise um eine sogenannte Oxacarbonsäure. Solche Oxacarbonsäuren (eng.: oxa acids) sind Verbindungen, die neben einer oder mehreren Carboxylgruppen zusätzlich Sauerstoffatome innerhalb der Restkette der Carbonsäure tragen. Entsprechende Verbindungen werden beispielsweise von der Clariant GmbH, Deutschland (Hoechst Fine Chemicals) angeboten und vertrieben.

Oxasäuren bzw. Oxacarbonsäuren liegen üblicherweise in Form von Mono- oder Dicarbonsäuren vor. Erfindungsgemäß ist die Verwendung von Oxamonocarbon-säuren bevorzugt.

Innerhalb des Kettenrests der Carbonsäure können bei Oxasäuren ein Sauerstoffatom oder auch zwei und mehrere Sauerstoffatome vorgesehen sein. Letztere bezeichnet man als Oligooxacarbonsäuren, deren Verwendung erfindungsgemäß ebenfalls bevorzugt ist.

Weiter bevorzugt sind Ausführungsformen, bei denen die Oxacarbonsäure 6 bis 12, insbesondere 8 bis 10 Kettenatome aufweist. Dabei werden die Kohlenstoffatome der Carboxylgruppe mitgezählt. Auch hier sind Oligooxacarbonsäuren mit den entsprechenden Kettenlängen bevorzugt, d. h. Oxasäuren mit mehreren Sauerstoffatomen innerhalb der Kette. Insbesondere können erfindungsgemäß Trioxacarbonsäuren als Dispergierungsmittel verwendet werden. Von diesen Trioxacarbonsäuren ist 3, 6, 9-Trioxadecansäure als besonders bevorzugt zu nennen. Es handelt sich dabei um eine Oxasäure mit einer 10er-Kette, wobei sich die drei Sauerstoffatome innerhalb der Kette an den Positionen 3, 6 und 9 (Position 1 ist das Kohlenstoffatom der Carboxylgruppe) befinden.

Bei der Erfindung kann die Menge an Dispergierungsmittel in der Suspension innerhalb weiter Grenzen variiert werden. Bevorzugt sind Mengen an Dispergierungsmittel zwischen 0,1 und 10 Gewichtsprozent, bezogen auf den Feststoffgehalt der Suspension. Innerhalb dieses Bereichs sind Mengen zwischen 0,1 und 2 Gewichtsprozent weiter bevorzugt.

Bei weiteren Ausführungsformen der Erfindung können innerhalb der Suspension mindestens 2 Fraktionen keramischer Partikel mit unterschiedlicher mittlerer Korngröße enthalten sein. Auf diese Weise kann erreicht werden, daß die Dichte des abgeschiedenen Grünkörpers erhöht wird, da die keramischen Partikel mit kleinerer mittlerer Korngröße die Zwischenräume zwischen den keramischen Partikeln mit größerer mittlerer Korngröße zumindest teilweise ausfüllen. Bekanntermaßen folgt die Korngrößenverteilung einer Fraktion keramischer Partikel mit bestimmter mittlerer Korngröße einer Gauss-Verteilung. Dementsprechend sind bei den beschriebenen bevorzugten Ausführungen (um in diesem Bild zu bleiben) die zwei oder mehr Gauss-Kurven gegeneinander verschoben.

Der keramische Grünkörper wird (vor oder nach seiner Bearbeitung) bei den Temperaturen gesintert, die sich aus den verwendeten Keramikmaterialien ergeben. Vorzugsweise liegt die Sintertemperatur zwischen 1100 °C und 1700 °C, insbesondere zwischen 1150 °C und 1500 °C. Vorzugsweise beträgt die Sintertemperatur ca. 1400 °C.

Die Sinterzeit wird ebenfalls z. B. in Abhängigkeit von dem verwendeten Keramikmaterial gewählt. Hier sind bevorzugte Sinterzeiten zwischen 2 und 10 Stunden, insbesondere zwischen 4 und 6 Stunden zu nennen. Bei weiteren bevorzugten Ausführungsformen wird ca. 5 Stunden gesintert.

Um eine homogene Temperaturverteilung im Grünkörper zu erreichen, wird dieser allmählich auf die endgültige Sintertemperatur gebracht. Bevorzugte Aufheizraten betragen hier zwischen 1 und 20 °C/min, insbesondere zwischen 5 und 10 °C/min. Innerhalb des zuletzt genannten Bereichs sind Aufheizraten zwischen 5 und 7,5 °C/min weiter bevorzugt.

Vorzugsweise wird im Sinterschritt bei der Erfindung so vorgegangen, daß das Arbeitsmodell zusammen mit dem darauf abgeschiedenen Grünkörper bei Raumtemperatur an Luft getrocknet und dann anschließend in den Ofen überführt wird. Dort wird das Arbeitsmodell zusammen mit dem Grünkörper bis auf 900 °C erhitzt, wobei hier eine vergleichsweise geringe Aufheizrate verwendet werden kann. Dieses Aufheizen kann stufenweise erfolgen, wobei Haltezeiten bei den entsprechenden Temperaturen vorgesehen sein können. Durch dieses Erhitzen wird der Grünkörper vorgesintert, wobei das Gipsmaterial des Arbeitsmaterials schrumpft, da der Gips sein Kristallwasser teilweise verliert. Dann wird das Arbeitsmodell zusammen mit dem Grünkörper kurz aus dem Ofen genommen und der Grünkörper vom Arbeitsmodell entformt. Dies geschieht leicht, da das Arbeitsmodell wie beschrieben geschrumpft ist. Dann wird der vorgesinterte Grünkörper, beispielsweise in Form eines Käppchens, wieder in den Ofen gegeben. Dann wird der Ofen, vorzugsweise mit einer vergleichsweise hohen Aufheizrate auf die endgültige Sintertemperatur gebracht und das Formteil fertig gesintert.

Nach dem Sinterschritt werden vollkeramische Formteile mit Dichten von mehr als 90 % der theoretischen Dichte, vorzugsweise mehr als 95 % der theoretischen Dichte bereitgestellt. Solche Vollkeramikteile, beispielsweise in Form eines Käppchens, können dann in üblicher Weise wie ein Metallkäppchen mit Verblendkeramik versehen und gebrannt werden. Auf diese Weise entsteht dann der endgültige Zahnersatz, der beispielsweise in Form einer Krone oder Brücke in den Mund des Patienten eingesetzt wird. Selbstverständlich kann erfindungs-gemäß herstellbarer Zahnersatz auch auf dentale Suprakonstruktionen, wie beispielsweise Implantatteile aufgesetzt werden.

Bei der Erfindung wird die Suspension keramischer Partikel, der sogenannte keramische Schlicker, üblicherweise auf ein Positivmodell der Grundstruktur, für die das Dentalformteil vorgesehen ist, d. h. im Normalfall auf ein Arbeitsmodell aufgebracht. Bei bevorzugten Ausführungsformen der Erfindung weist dieses Positivmodell/Arbeitsmodell größere Abmessungen/Dimensionen auf als die Grünstruktur. Durch diese größeren Abmessungen wird der im Sinterschritt eintretende Sinterschrumpf bereits vorab kompensiert. Nach Abschluß des Sinterschritts wird dementsprechend ein Dentalformteil, beispielsweise ein Vollkeramik-Käppchen erhalten, das exakt auf die Grundstruktur, d. h. beispielsweise den präparierten Zahnstumpf paßt.

Die größeren Abmessungen des Positivmodells können auf unterschiedliche Weise erreicht werden. So ist es insbesondere möglich, das Positivmodell aus einem Material herzustellen, das eine ausreichende und gegebenenfalls exakt eingestellte Abbindeexpansion aufweist. So liegen üblicherweise Volumenexpansionen von Dentalgipsen im Bereich von weniger als 0,1 % bis etwas 0,5 %. Erfindungsgemäß ist es dementsprechend möglich, Materialien, insbesondere auf Basis von Gips zu verwenden, die beim Abbinden Volumenausdehnungen zwischen 1 % und 25 %, vorzugsweise zwischen 3 % und 10 % besitzen.

Bei weiteren bevorzugten Ausführungsformen des erfindungsgemäßen Verfahrens kann ein Negativmodell, mit dessen Hilfe ein Positivmodell/Arbeitsmodell her-gestellt wird, größere Abmessungen als die Grundstruktur besitzen. Insbesondere kann es sich bei dem Negativmodell um den beim Duplierschritt hergestellten Abdruck des Meistermodells handeln.

Auch bei den zuletzt beschriebenen Ausführungsformen können die größeren Abmessungen/Dimensionen des Negativmodells grundsätzlich auf verschiedene Weise bereitgestellt werden. Insbesondere wird zur Herstellung des Negativmodells ein Material, insbesondere elastisches Kunststoffmaterial, verwendet, das eine ausreichende Abbindeexpansion oder sogar einen Abbindeschrumpf aufweist. Es ist auch möglich, ein Material nach dem Abbinden nachträglich zu expandieren, beispielsweise durch Quellung in einem Lösungsmittel. Es ist bevorzugt, wenn die Volumenänderung des Materials zwischen 1 % und 25 %, insbesondere zwischen 3 % und 10 % beträgt.

Bei allen beschriebenen bevorzugten Ausführungsformen können die jeweiligen Schritte zur Herstellung des Positivmodells oder Negativmodells mehrmalig durchgeführt werden. Auf diese Weise kann man sich den gewünschten größten Abmessungen zur Kompensation des Sinterschrumpfs sukzessive annähern oder gegebenenfalls sogar verschiedene vollkeramische Formteile erstellen und deren Passung mit der Grundstruktur testen.

Für den Fall, daß das Formteil im Mund unlösbar mit der Grundstruktur verbunden werden soll (nicht-herausnehmbarer Zahnersatz) kann bei der Herstellung des Positivmodells oder des Negativmodells mit den größeren Abmessungen auch die Schichtdicke des Klebers oder Zahnzements, die beim Einbringen des Formteils in den Mund notwendig sind, berücksichtigt werden. Dies ist selbstverständlich im Falle von herausnehmbarem Zahnersatz nicht erforderlich.

Weiter ist es erfindungsgemäß möglich, das Positivmodell oder auch das Negativmodell mit den entsprechenden größeren Abmessungen aus Vollmaterial herauszuarbeiten. Dies kann vorzugsweise durch das sogenannte CADCAM-Fräsen erfolgen. In diesen Fällen wird das überdimensionierte Arbeitsmodell oder der überdimensionierte Duplierabdruck durch digitales Scannen der Original-situation, durch rechnerisches Bearbeiten des erhaltenen Datensatzes unter Einbringung eines Korrekturfaktors für den zu erwartenden Sinterschrumpf und anschließende digital unterstützte Herstellung des Modells bereitgestellt.

Eine weitere bevorzugte Möglichkeit zur Herstellung des Positivmodells/Negativmodells mit den entsprechenden größeren Abmessungen ist das sogenannte Rapid Prototyping. Bei diesem grundsätzlich bekannten Verfahren wird vorzugsweise eine Kunststoffmonomerlösung mit Laserlicht polymerisiert und zwar exakt in der vorgegebenen Form, die in ganz ähnlicher Weise wie oben be-schrieben digital erfaßt ist.

In der Zeichnung zeigt:

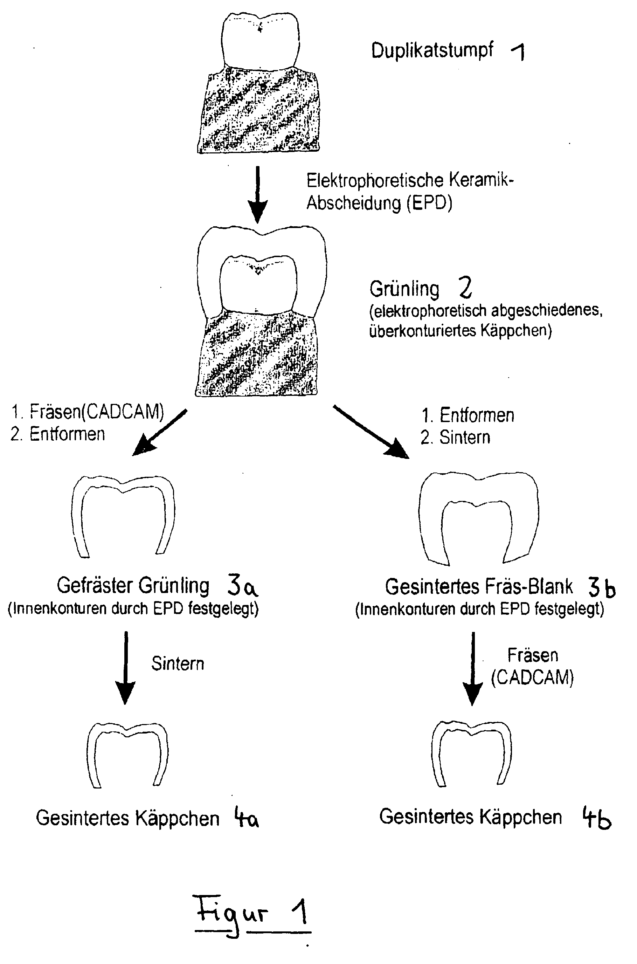

Fig. 1    den schematischen Verfahrensablauf für zwei bevorzugte Ausführungen des erfindungsgemäßen Verfahrens.

Gemäß Fig. 1 geht das erfindungsgemäße Verfahren von einem Duplikatstumpf 1 aus, der in üblicher Weise als Arbeitsmodell von einem Zahnstumpf präpariert wurde. Der Duplikatstumpf 1 kann vorzugsweise etwas größere Dimensionen als der Zahnstumpf aufweisen, um den Sinterschrumpf des später aufgebrachten keramischen Materials auszugleichen.

Auf den Duplikatstumpf 1 wird dann, wie beschrieben, durch elektrophoretische Abscheidung (EPD) eine Schicht keramischer Partikel aufgebracht. Auf diese Weise entsteht der Grünling 2 in Form eines elektrophoretisch abgeschiedenen Käppchens. Dieser Grünling 2 besitzt eine vergleichsweise große Dicke und ist überkonturiert, um die nachfolgende Bearbeitung (unter Abtrag des Keramikmaterials) zu ermöglichen.

Ausgehend vom Grünling 2 sind in Fig. 1 zwei bevorzugte alternative Verfahrensabläufe dargestellt.

Gemäß der ersten (linken) Alternative wird der sich noch auf dem Duplikatstumpf 1 (Arbeitsmodell) befindliche Grünling 2 (CADCAM-gestützt) gefräst. Anschließend wird der Grünling 2 vom Duplikatstumpf 1 entformt/entfernt und liegt dann in Form des gefrästen Grünlings 3 a vor. Bei diesem gefrästen Grünling 3 a sind die Innenkonturen (inneren Oberflächen) durch die elektrophoretische Abscheidung fest-gelegt. Die Außenkonturen sind durch den Fräsvorgang festgelegt. Anschließend wird bei dieser Alternative der gefräste Grünling 3 a zu dem fertig gesinterten Käppchen 4 a gesintert.

Gemäß der in Fig. 1 darstellten zweiten (rechten) Alternative wird der Grünling 2 vom Duplikatstumpf 1 (Arbeitsmodell) entformt/entfernt und dann gesintert. Der auf diese Weise erhaltene sogenannte gesinterte Fräs-Blank 3 b wird anschließend durch auf CADCAM-gestütztes Fräsen zu dem gesinterten Käppchen 4 b bearbeitet. Bei diesem gesinterten Käppchen 4 b sind die Innenkonturen (innere Oberflächen) durch die elektrophoretische Abscheidung festgelegt und die Außenkonturen durch den Fräsvorgang.

Es ist offensichtlich, daß die Erfindung nicht auf die beiden dargestellten Ausführungen des Verfahrens beschränkt ist. Kern der Erfindung ist, daß ein formgebendes Verfahren, insbesondere die elektrophoretische Abscheidung zur Herstellung der inneren Oberflächen des Dentalformteils und ein Bearbeitungsschritt unter Abtrag des Keramikmaterials zur Herstellung der äußeren Oberflächen des Dentalteils angewendet wird. Solange diese Verfahrensschritte erhalten bleiben, kann das erfindungsgemäße Verfahren beliebig abgewandelt werden. So kann beispielsweise bei der in Fig. 1 dargestellten rechten Alternative der Grünling 2 vor dem Fräsen nur teilweise gesintert werden und das erhaltene Dentalformteil nach dem Fräsen fertiggesintert werden.

**Beispiel**

Schlickerherstellung mit Zirkondioxid-Pulver

Es werden zu 100 g Ethanol, in dem zuvor 0,8 g 3,6,9-Trioxadecansäure mit Hilfe eines Magnetrührers gelöst wurden, portionsweise 100 g ZirkonoxidPulver unter Rühren zugegeben. Die Primärpartikelgröße (Partikelgröße in nicht-agglomerier-tem Zustand) des hierbei verwendeten Zirkondioxid-Pulvers liegt bei ca. 0,6 $\mu$m. Zur vollständigen Deagglomeration der hergestellten Suspension erfolgt an-schließend eine 5 Minuten andauernde Ultraschallbehandlung. Der resultierenden Suspension werden 5 g Polyvinylbutyral zugesetzt. Eine Homogenisierung des erhaltenen Schlickers erfolgt erneut durch eine Ultraschallbehandlung.

Verwendete Chemikalien: Zirkonoxidpulver SC 15 (Fa. MEL CHEMICALS); Trioxadecansäure (Fa. CLARIANT); Polyvinylbutyral, Molekulargewicht 70.000 (Fa. CLARIANT).

**Herstellung der vollkeramischen Dentalformteile**

Vom Meistermodell einer Einzelzahnpräparation wird mit Hilfe eines Duplier-Negativmodells ein Arbeitsmodell (Positivmodell) hergestellt. Zu diesem Zweck wird das Negativmodell mit einem Gipsmaterial ausgegossen, das beim Abbinden eine Volumenausdehnung von ca. 10 % aufweist. Das Material des Negativmodells läßt aufgrund seiner Elastizität die größere Expansion des Gipses zu. Die erhöhte Expansion ist derart eingestellt, daß sie den Sinterschrumpf eines gemäß der obigen Vorschrift hergestellten Schlickers aus ZirkonoxidPulver kompensiert.

Der als Arbeitsmodell dienende Gipsstumpf wird mit Leitsilberlack bestrichen und zusammen mit einer Platin-Gegenelektrode in üblicher Weise in den Schlicker eingetaucht. An beiden Elektroden wird eine konstante Spannung von ca. 30 V angelegt. Hier sind bevorzugte Bereiche von 5 V bis 100 V, vorzugsweise zwischen 10 V und 30 V möglich. Es kann auch so verfahren werden, daß ein konstanter Strom von 10 mA angelegt wird. Hier sind bevorzugte Bereiche zwischen 0,1 mA und 100 mA, vorzugsweise zwischen 0,2 mA und 10 mA, möglich. Als Abscheidedauer wird im vorliegenden Fall ein Zeitraum von 20 Minuten gewählt. Hier können je nach gewünschter Schichtdicke Abscheide-dauern zwischen 1 Minute und 60 Minuten, vorzugsweise zwischen 5 Minuten und 20 Minuten, gewählt werden. Im vorliegenden Fall wurde die Abscheidung unter leichtem Rühren durchgeführt, wobei auf diesem Rührvorgang gegebenenfalls auch verzichtet werden kann.

Durch diese elektrophoretische Abscheidung wird ein keramischer Grünkörper erhalten, der zusammen mit dem Arbeitsmodell getrocknet wird. Dann wird in der bereits oben beschriebenen Weise vorgesintert (Aufheizen mit 2 °C/min auf 600 °C; ½ Stunde bei 600 °C halten; dann Aufheizen mit 5 °C/min auf 900 °C; dann 1 Stunde bei 900 °C halten) und das Arbeitsmodell vom vorgesinterten Grünkörper entformt. Dieser wird anschließend bei 1400 °C knapp 5 Stunden lang gesintert. Es wird eine Aufheizrate von 15 °C/min. gewählt, um den vorgesinterten Grünkörper auf die endgültige Sintertemperatur zu bringen.

Das so erhaltene Vollkeramikkäppchen paßt mit hoher Genauigkeit auf das Meistermodell und dementsprechend auch auf die Zahnpräparation. Die Dichte des Käppchens liegt bei > 90 % der theoretischen Dichte. Außerdem weist das Käppchen keinerlei Risse auf und besitzt eine ansprechende weiße Farbe mit einer gewissen Transluzens.

Das auf diese Weise erhaltene Vollkeramikkäppchen wird anschließend durch CADCAM-gestütztes Fräsen nachbearbeitet, d.h. seine äußeren Oberflächen werden unter Abtrag von Keramikmaterial gefräst, um eine entsprechende vorgegebene Außenkontur zu erhalten. Die so erhaltene Außenoberfläche läßt sich anschließend verblenden, beispielsweise mit Kunststoffmaterial oder vorzugsweise mit Verblendkeramiken.

Es wird nochmals darauf hingewiesen, daß die Erfindung nicht auf das beschriebene Beispiel beschränkt ist. Im Beispiel wird ein bereits gesintertes Keramikkäppchen gefräst, was der rechten Verfahrensalternative in Fig. 1 entspricht. Selbstverständlich kann dieses Verfahren im Rahmen der Erfindung entsprechend abgewandelt werden.

**Patentansprüche**

1. Verfahren zur Herstellung vollkeramischer Dentalformteile wie Kronen, Brücken o. dgl., bei dem

   - eine Suspension keramischer Partikel auf ein Modell der Grundstruktur, für die das Dentalformteil vorgesehen ist, aufgebracht wird, und
   - mindestens die äußere, dem Modell abgewandte Oberfläche des erhaltenen keramischen Körpers, gegebenenfalls nach Entfernen von dem Modell, mindestens teilweise unter Abtrag von keramischem Material bearbeitet wird,

   **dadurch gekennzeichnet, dass**

- die keramischen Partikel durch elektrophoretische Abscheidung auf das Modell aufgebracht werden.

2. Verfahren nach Anspruch 1, **dadurch gekennzeichnet, dass** die Bearbeitung spanabhebend erfolgt.

3. Verfahren nach Anspruch 1 oder 2, **dadurch gekennzeichnet, dass** die Bearbeitung durch Fräsen erfolgt.

4. Verfahren nach einem der vorhergehenden Ansprüche, **dadurch gekennzeichnet, dass** die Bearbeitung CADCAM-gestützt erfolgt.

5. Verfahren nach einem der vorhergehenden Ansprüche, **dadurch gekennzeichnet, dass** der durch das Aufbringen der keramischen Partikel erhaltene keramische Körper im sogenannten Grünzustand bearbeitet wird.

6. Verfahren nach einem der Ansprüche 1 bis 4, **dadurch gekennzeichnet, dass** der durch das Aufbringen der keramischen Partikel erhaltene keramische Körper in einem mindestens teilweise gesinterten Zustand bearbeitet wird.

7. Verfahren nach einem der vorhergehenden Ansprüche, **dadurch gekennzeichnet, dass** die Abmessungen des Modells für die Herstellung des keramischen Körpers so gewählt sind, dass der beim Sintern dieses Körpers eintretende Schrumpf kompensiert wird, um die gewünschte Passung zwischen Dentalformteil und Grundstruktur zu erreichen.

8. Verfahren nach einem der vorhergehenden Ansprüche, **dadurch gekennzeichnet, dass** der keramische Körper nach der Bearbeitung gesintert wird.

9. Verfahren nach einem der vorhergehenden Ansprüche, **dadurch gekennzeichnet, dass** der keramische Körper nach der Bearbeitung im gesinterten Zustand verblendet wird, wobei es sich bei der Verblendung vorzugsweise um eine keramische Verblendung handelt.

10. Verfahren nach einem der vorhergehenden Ansprüche, **dadurch gekennzeichnet, dass** es sich bei den keramischen Partikeln mindestens teilweise um keramische Fasern und/oder nanokristalline Partikel handelt.

11. Verfahren nach Anspruch 10, **dadurch gekennzeichnet, dass** der Anteil der keramischen Fasern an der Gesamtmenge der keramischen Partikel zwischen 0,5 Gewichtsprozent und 5 Gewichtsprozent, insbesondere zwischen 1 Gewichtsprozent und 2 Gewichtsprozent beträgt.

12. Verfahren nach Anspruch 10 oder Anspruch 11, **dadurch gekennzeichnet, dass** der Anteil der nanokristallinen Partikel an der Gesamtmenge der keramischen Partikel zwischen 0,5 Gewichtsprozent und 10 Gewichtsprozent, insbesondere zwischen 1 Gewichtsprozent und 2 Gewichtsprozent beträgt.

13. Verfahren nach einem der vorhergehenden Ansprüche, **dadurch gekennzeichnet, dass** in der Suspension zusätzlich mindestens ein metallisches Pulver enthalten ist.

14. Verfahren nach Anspruch 13, **dadurch gekennzeichnet, dass** der Anteil an metallischem Pulver an der Gesamtmenge der in der Suspension enthaltenen Partikel zwischen 10 Gewichtsprozent und 80 Gewichtsprozent, insbesondere zwischen 30 Gewichtsprozent und 60 Gewichtsprozent beträgt.

15. Verfahren nach einem der vorhergehenden Ansprüche, **dadurch gekennzeichnet, dass** die Suspension mindestens ein Dispergierungsmittel und gegebenenfalls weitere Additive, beispielsweise Bindemittel, enthält.

16. Verfahren nach Anspruch 15, **dadurch gekennzeichnet, dass** es sich bei dem Dispergierungsmittel um eine Carbonsäure, vorzugsweise um eine Oxacarbonsäure, insbesondere um eine O-xamonocarbonsäure handelt.

17. Verfahren nach Anspruch 16, **dadurch gekennzeichnet, dass** es sich bei dem Dispergierungsmittel um eine Oligooxacarbonsäure handelt.

18. Verfahren nach Anspruch 17, **dadurch gekennzeichnet, dass** es sich um eine Oxacarbonsäure, insbesondere um eine Oligooxacarbonsäure mit 6 bis 12, vorzugsweise 8 bis 10 Kettenatomen handelt.

19. Verfahren nach einem der Ansprüche 16 bis 18, **dadurch gekennzeichnet, dass** es sich bei dem Dispergierungsmittel um eine Trioxacarbonsäure, vorzugsweise 3,6,9-Trioxadecansäure handelt.

20. Verfahren nach einem der Ansprüche 15 bis 19, **dadurch gekennzeichnet, dass** das Dispergierungsmittel in der Suspension in einer Menge zwischen 0,1 und 10 Gewichtsprozent, vorzugsweise zwischen 0,1 und 2 Gewichtsprozent, bezogen auf den Feststoffgehalt der Suspension, enthalten ist.

21. Verfahren nach einem der vorhergehenden Ansprüche, **dadurch gekennzeichnet, dass** die Suspension ein Zeta-Potential zwischen $\pm$ 1 mV und $\pm$ 100 mV, vorzugsweise zwischen $\pm$ 30 mV und $\pm$ 50 mV

besitzt.

**22.** Verfahren nach einem der vorhergehenden Ansprüche, **dadurch gekennzeichnet, dass** die Suspension ein polares Lösungsmittel aufweist.

**23.** Verfahren nach Anspruch 22, **dadurch gekennzeichnet, dass** es sich bei dem Lösungsmittel um Wasser handelt.

**24.** Verfahren nach Anspruch 22, **dadurch gekennzeichnet, dass** es sich bei dem Lösungsmittel um einen Alkohol, vorzugsweise um einen aliphatischen Alkohol, insbesondere Ethanol handelt.

**25.** Verfahren nach einem der vorhergehenden Ansprüche, **dadurch gekennzeichnet, dass** es sich bei den keramischen Partikeln um oxidkeramische Partikel, vorzugsweise um Aluminiumoxid-Partikel und/ oder Zirkonoxid-Partikel handelt.

**26.** Verfahren nach einem der vorhergehenden Ansprüche, **dadurch gekennzeichnet, dass** in der Suspension mindestens zwei Fraktionen keramischer Partikel mit unterschiedlicher mittlerer Korngröße enthalten sind.

**27.** Verfahren nach einem der vorhergehenden Ansprüche, **dadurch gekennzeichnet, dass** die Korngrößen der keramischen Partikel zwischen 1 nm und 100 um, vorzugsweise zwischen 100 nm und 10 um liegen.

**28.** Verfahren nach einem der vorhergehenden Ansprüche, **dadurch gekennzeichnet, dass** die keramischen Partikel in der Suspension in einer Menge zwischen 10 und 90 Gewichtsprozent, vorzugsweise zwischen 40 und 60 Gewichtsprozent, bezogen auf das Gesamtgewicht der Suspension, enthalten sind.

**29.** Verfahren nach einem der Ansprüche 15 bis 28, **dadurch gekennzeichnet, dass** es sich bei dem Bindemittel um mindestens einen Polyvinylalkohol oder um mindestens ein Polyvinlybutyral handelt.

**30.** Verfahren nach einem der Ansprüche 15 bis 29, **dadurch gekennzeichnet, dass** das Bindemittel in der Suspension in einer Menge zwischen 0,1 bis 20 Gewichtsprozent, vorzugsweise 0,2 bis 10 Gewichtsprozent, bezogen auf den Feststoffgehalt der Suspension, enthalten ist.

**31.** Verfahren nach einem der Ansprüche 6 bis 30, **dadurch gekennzeichnet, dass** der keramische Körper bei einer Temperatur zwischen 1100 °C und 1700 °C, vorzugsweise zwischen 1150 °C und 1500 °C, insbesondere bei ca. 1400 °C gesintert wird.

**32.** Verfahren nach einem der Ansprüche 6 bis 31, **dadurch gekennzeichnet, dass** der keramische Körper zwischen 2 und 10 Stunden, vorzugsweise zwischen 4 und 6 Stunden, insbesondere ca. 5 Stunden gesintert wird.

**33.** Verfahren nach einem der Ansprüche 6 bis 32, **dadurch gekennzeichnet, dass** der keramische Körper mit einer Aufheizrate zwischen 1 und 20 °C/min, vorzugsweise 5 und 10 °C/min, insbesondere zwischen 5 und 7,5 °C/min auf die Sintertemperatur gebracht wird.

**34.** Verfahren nach einem der Ansprüche 6 bis 33, **dadurch gekennzeichnet, dass** der keramische Körper zusammen mit dem Modell, vorzugsweise nach Trocknung bei Raumtemperatur an Luft, zunächst mit einer vergleichsweise geringen Aufheizrate auf eine erste Temperatur, insbesondere ca. 900 °C erhitzt und dann mit einer höheren Aufheizrate auf eine zweite Temperatur, die höher ist als die erste Temperatur, insbesondere ca. 1400 °C, erhitzt und fertig gesintert wird.

**Claims**

**1.** Method for production of fully ceramic dental mouldings such as crowns, bridges or the like, in which method

- a suspension of ceramic particles is applied to a model of the base structure for which the dental moulding is provided, and
- at least the outer surface of the resulting ceramic body directed away from the model is machined with at least partial removal of ceramic material, if appropriate after removal from the model,

**characterized in that**

- the ceramic particles are applied to the model by electrophoretic deposition.

**2.** Method according to Claim 1, **characterized in that** the machining is carried out by cutting.

**3.** Method according to Claim 1 or 2, **characterized in that** the machining is carried out by milling.

**4.** Method according to one of the preceding claims, **characterized in that** the machining is CAD/CAM machining.

**5.** Method according to one of the preceding claims, **characterized in that** the ceramic body obtained by application of the ceramic particles is machined in the so-called green state.

**6.** Method according to one of Claims 1 to 4, **characterized in that** the ceramic body obtained by application of the ceramic particles is machined in an at least partially sintered state.

**7.** Method according to one of the preceding claims, **characterized in that** the dimensions of the model for the production of the ceramic body are chosen such that the shrinkage occurring during sintering of this body is compensated, in order to achieve the desired fit between dental moulding and base structure.

**8.** Method according to one of the preceding claims, **characterized in that** the ceramic body is sintered after the machining.

**9.** Method according to one of the preceding claims, **characterized in that** the ceramic body is veneered in the sintered state after the machining, the veneer preferably being a ceramic veneer.

**10.** Method according to one of the preceding claims, **characterized in that** at least some of the ceramic particles are ceramic fibres and/or nanocrystalline particles.

**11.** Method according to Claim 10, **characterized in that** the proportion of the ceramic fibres, based on the total quantity of the ceramic particles, is between 0.5 percent by weight and 5 percent by weight, in particular between 1 percent by weight and 2 percent by weight.

**12.** Method according to Claim 10 or Claim 11, **characterized in that** the proportion of the nanocrystalline particles, based on the total quantity of the ceramic particles, is between 0.5 percent by weight and 10 percent by weight, in particular between 1 percent by weight and 2 percent by weight.

**13.** Method according to one of the preceding claims, **characterized in that** at least one metal powder is additionally contained in the suspension.

**14.** Method according to Claim 13, **characterized in that** the proportion of metal powder, based on the total quantity of the particles contained in the suspension, is between 10 percent by weight and 80 percent by weight, in particular between 30 percent by weight and 60 percent by weight.

**15.** Method according to one of the preceding claims, **characterized in that** the suspension contains at least one dispersing agent and, if appropriate, further additives, for example binders.

**16.** Method according to Claim 15, **characterized in that** the dispersing agent is a carboxylic acid, preferably an oxacarboxylic acid, in particular an oxamonocarboxylic acid.

**17.** Method according to Claim 16, **characterized in that** the dispersing agent is an oligooxacarboxylic acid.

**18.** Method according to Claim 17, **characterized in that** it involves an oxacarboxylic acid, in particular an oligooxacarboxylic acid with 6 to 12, preferably 8 to 10 chain atoms.

**19.** Method according to one of Claims 16 to 18, **characterized in that** the dispersing agent is a trioxacarboxylic acid, preferably 3,6,9-trioxadecanoic acid.

**20.** Method according to one of Claims 15 to 19, **characterized in that** the dispersing agent is contained in the suspension in a quantity of between 0.1 and 10 percent by weight, preferably between 0.1 and 2 percent by weight, based on the solids content of the suspension.

**21.** Method according to one of the preceding claims, **characterized in that** the suspension has a zeta potential of between $\pm$ 1 mV and $\pm$ 100 mV, preferably between $\pm$ 30 mV and $\pm$ 50 mV.

**22.** Method according to one of the preceding claims, **characterized in that** the suspension comprises a polar solvent.

**23.** Method according to Claim 22, **characterized in that** the solvent is water.

**24.** Method according to Claim 22, **characterized in that** the solvent is an alcohol, preferably an aliphatic alcohol, in particular ethanol.

**25.** Method according to one of the preceding claims, **characterized in that** the ceramic particles are oxide-ceramic particles, preferably aluminium oxide particles and/or zirconium oxide particles.

**26.** Method according to one of the preceding claims, **characterized in that** at least two fractions of ceramic particles with different mean particle size are contained in the suspension.

**27.** Method according to one of the preceding claims, **characterized in that** the sizes of the ceramic particles are between 1 nm and 100 $\mu$m, preferably between 100 nm and 10 $\mu$m.

**28.** Method according to one of the preceding claims, **characterized in that** the ceramic particles are contained in the suspension in a quantity of between 10 and 90 percent by weight, preferably between 40 and 60 percent by weight, based on the total weight of the suspension.

29. Method according to one of Claims 15 to 28, **characterized in that** the binder is at least one polyvinyl alcohol or at least one polyvinyl butyral.

30. Method according to one of Claims 15 to 29, **characterized in that** the binder is contained in the suspension in a quantity of between 0.1 and 20 percent by weight, preferably 0.2 to 10 percent by weight, based on the solids content of the suspension.

31. Method according to one of Claims 6 to 30, **characterized in that** the ceramic body is sintered at a temperature of between 1100°C and 1700°C, preferably between 1150°C and 1500°C, in particular at ca. 1400°C.

32. Method according to one of Claims 6 to 31, **characterized in that** the ceramic body is sintered for between 2 and 10 hours, preferably between 4 and 6 hours, in particular ca. 5 hours.

33. Method according to one of Claims 6 to 32, **characterized in that** the ceramic body is brought to the sintering temperature at a heating rate of between 1 and 20°C/min, preferably between 5 and 10°C/min, in particular between 5 and 7.5°C/min.

34. Method according to one of Claims 6 to 33, **characterized in that** the ceramic body, together with the model, preferably after drying at room temperature in air, is firstly heated at a comparatively low heating rate to a first temperature, in particular ca. 900°C, and is then heated at a higher heating rate to a second temperature, which is higher than the first temperature, in particular ca. 1400°C, and is finally sintered.

**Revendications**

1. Procédé de fabrication de pièces dentaires entièrement céramiques, telles que des couronnes, des bridges ou analogues, dans lequel

   - on applique une suspension de particules céramiques sur un modèle de la structure de base pour laquelle est prévue la pièce dentaire, et
   - on usine au moins partiellement, par enlèvement de matière céramique, la surface extérieure, opposée au modèle, du corps céramique ainsi obtenu, éventuellement après qu'il a été enlevé du modèle,

**caractérisé en ce que**

   - les particules céramiques sont appliquées sur le modèle par dépôt électrophorétique.

2. Procédé selon la revendication 1, **caractérisé en ce que** l'usinage est effectué par enlèvement de copeaux.

3. Procédé selon la revendication 1 ou 2, **caractérisé en ce que** l'usinage est effectué par fraisage.

4. Procédé selon l'une des revendications précédentes, **caractérisé en ce que** l'usinage est effectué en CFAO.

5. Procédé selon l'une des revendications précédentes, **caractérisé en ce que** le corps céramique obtenu après application des particules céramiques est usiné à ce que l'on appelle l'état vert.

6. Procédé selon l'une des revendications 1 à 4, **caractérisé en ce que** le corps céramique obtenu par application des particules céramiques est usiné dans un état au moins partiellement fritté.

7. Procédé selon l'une des revendications précédentes, **caractérisé en ce que** les dimensions du modèle destiné à la fabrication du corps céramique sont choisies de façon à compenser le retrait apparaissant lors du frittage de ce corps, pour réaliser l'adaptation souhaitée entre la pièce dentaire et la structure de base.

8. Procédé selon l'une des revendications précédentes, **caractérisé en ce que** le corps céramique est fritté après usinage.

9. Procédé selon l'une des revendications précédentes, **caractérisé en ce que** le corps céramique est facetté après usinage à l'état fritté, le facettage étant de préférence un facettage céramique.

10. Procédé selon l'une des revendications précédentes, **caractérisé en ce que** les particules céramiques sont au moins partiellement des fibres céramiques et/ou des particules nanocristallines.

11. Procédé selon la revendication 10, **caractérisé en ce que** la proportion des fibres céramiques, par rapport à la quantité totale des particules céramiques, est comprise entre 0,5 et 5 % en poids, en particulier entre 1 et 2 % en poids.

12. Procédé selon la revendication 10 ou 11, **caractérisé en ce que** la proportion des particules nanocristallines, par rapport à la quantité totale des particules céramiques, est comprise entre 0,5 et 10 % en poids, en particulier entre 1 et 2 % en poids.

13. Procédé selon l'une des revendications précédentes, **caractérisé en ce que** la suspension contient en outre au moins une poudre métallique.

14. Procédé selon la revendication 13, **caractérisé en ce que** la proportion de la poudre métallique, par rapport à la quantité totale des particules contenues dans la suspension, est comprise entre 10 et 80 % en poids, en particulier entre 30 et 60 % en poids.

15. Procédé selon l'une des revendications précédentes, **caractérisé en ce que** la suspension contient au moins un dispersant et éventuellement d'autres additifs, par exemple des liants.

16. Procédé selon la revendication 15, **caractérisé en ce que** le dispersant est un acide carboxylique, de préférence un acide oxacarboxylique, en particulier un acide oxamonocarboxylique.

17. Procédé selon la revendication 16, **caractérisé en ce que** le dispersant est un acide oligooxacarboxylique.

18. Procédé selon la revendication 17, **caractérisé en ce qu'**il s'agit d'un acide oxacarboxylique, en particulier d'un acide oligooxacarboxylique ayant 6 à 12 et de préférence 8 à 10 atomes dans la chaîne.

19. Procédé selon l'une des revendications 16 à 18, **caractérisé en ce que** le dispersant est un acide trioxacarboxylique, de préférence l'acide 3,6,9-trioxadécanoique.

20. Procédé selon l'une des revendications 15 à 19, **caractérisé en ce que** le dispersant est contenu dans la suspension en une quantité de 0,1 à 10 % en poids, de préférence de 0,1 à 2 % en poids par rapport à l'extrait sec de la suspension.

21. Procédé selon l'une des revendications précédentes, **caractérisé en ce que** la suspension possède un potentiel zêta compris entre $\pm$ 1 mV et $\pm$ 100 mV, de préférence entre $\pm$ 30 mV et $\pm$ 50 mV.

22. Procédé selon l'une des revendications précédentes, **caractérisé en ce que** la suspension comprend un solvant polaire.

23. Procédé selon la revendication 22, **caractérisé en ce que** le solvant est l'eau.

24. Procédé selon la revendication 22, **caractérisé en ce que** le solvant est un alcool, de préférence un alcool aliphatique, en particulier l'éthanol.

25. Procédé selon l'une des revendications précédentes, **caractérisé en ce que** les particules céramiques sont des particules céramiques à base d'oxydes, de préférence des particules d'oxyde d'aluminium et/ou des particules d'oxyde de zirconium.

26. Procédé selon l'une des revendications précédentes, **caractérisé en ce que** la suspension contient au moins deux fractions de particules céramiques ayant des granulométries moyennes différentes.

27. Procédé selon l'une des revendications précédentes, **caractérisé en ce que** la granulométrie des particules céramiques est comprise entre 1 nm et 100 $\mu$m, de préférence entre 100 nm et 10 $\mu$m.

28. procédé selon l'une des revendications précédentes, **caractérisé en ce que** les particules céramiques sont contenues dans la suspension en une quantité de 10 à 90 % en poids, de préférence de 40 à 60 % en poids par rapport au poids total de la suspension.

29. Procédé selon l'une des revendications 15 à 28, **caractérisé en ce que** le liant est au moins un poly (alcool vinylique) ou au moins un polyvinylbutyral.

30. Procédé selon l'une des revendications 15 à 29, **caractérisé en ce que** le liant est contenu dans la suspension en une quantité de 0,1 à 20 % en poids, de préférence de 0,2 à 10 en poids, par rapport à l'extrait sec de la suspension.

31. Procédé selon l'une des revendications 6 à 30, **caractérisé en ce que** le corps céramique est fritté à une température comprise entre 1 100 et 1 700°C, de préférence entre 1 150 et 1 500°C, en particulier à environ 1 400°C.

32. Procédé selon l'une des revendications 6 à 31, **caractérisé en ce que** le corps céramique est fritté pendant 2 à 10 heures, de préférence pendant 4 à 6 heures et en particulier pendant environ 5 heures.

33. Procédé selon l'une des revendications 6 à 32, **caractérisé en ce que** le corps céramique est porté à la température de frittage à une vitesse de montée en température de 1 à 20°C/min, de préférence de 5 à 10°C/min, en particulier de 5 à 7,5°C/min.

34. Procédé selon l'une des revendications 6 à 33, **caractérisé en ce que** le corps céramique, en même temps que le modèle, est, de préférence après séchage à l'air à la température ambiante, chauffé d'abord à une première température, en particulier d'environ 900°C, à une vitesse de montée en température relativement faible, puis est chauffé, à une vitesse de montée en température plus élevée, jusqu'à une deuxième température plus élevée que la première température, en particulier environ 1 400°C, et subit un frittage final.

Duplikatstumpf 1

Elektrophoretische Keramik-Abscheidung (EPD)

Grünling 2
(elektrophoretisch abgeschiedenes, überkonturiertes Käppchen)

1. Fräsen(CADCAM)
2. Entformen

1. Entformen
2. Sintern

Gefräster Grünling 3a
(Innenkonturen durch EPD festgelegt)

Gesintertes Fräs-Blank 3b
(Innenkonturen durch EPD festgelegt)

Sintern

Fräsen
(CADCAM)

Gesintertes Käppchen 4a

Gesintertes Käppchen 4b

Figur 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19816546 C1
- WO 9935994 A1
- WO 9950480 A1
- WO 0112097 A1